# EUROPÄISCHE PATENTANMELDUNG

(11) **EP 2 594 227 A2**
(43) Veröffentlichungstag der Anmeldung: **22.05.2013**
(21) Anmeldenummer: 12192519.2
(22) Anmeldetag: 14.11.2012
(51) Int. Cl.: A61C 9/00

(54) **Abformvorrichtung und Verfahren zur dreidimensionalen Erfassung von intraoralen Strukturen sowie eine entsprechende Einscanvorrichtung**

(30) Priorität: 15.11.2011 DE 102011055356
(71) Anmelder: Ludwig-Maximilians-Universität München, 80539 München (DE)
(72) Erfinder: Schweiger, Josef, 83346 Bergen (DE)
(74) Vertreter: Lucke, Andreas

(57) **Zusammenfassung**

Die Erfindung betrifft eine Abformvorrichtung (1) und ein Verfahren zur dreidimensionalen Erfassung von intraoralen Strukturen sowie eine entsprechende Einscanvorrichtung. Die erfindungsgemäße Abformvorrichtung (1) umfasst eine Abformhülse (2), die ein offenes und ein geschlossenes Ende aufweist, und mindestens einen Marker (3), der dreidimensional ausgebildet und außenseitig an der Abformhülse (2) angeordnet ist.

## Beschreibung

### GEBIET DER ERFINDUNG

Die Erfindung betrifft eine Abformvorrichtung und ein Verfahren zur dreidimensionalen Erfassung von intraoralen Strukturen, insbesondere von Zähnen und beschliffenen Zahnstümpfen, sowie eine entsprechende Einscanvorrichtung.

### VERWANDTER STAND DER TECHNIK

Auf dem Gebiet der Abformung der intraoralen Strukturen eines Patienten wird im Stand der Technik zwischen der analogen Erfassung mit Hilfe eines Abdrucks und der digitalen Erfassung mittels 3D-Erfassungsgeräten unterschieden. Eine Kombination beider Verfahren stellt der sogenannte Abdruckscan dar, bei dem ein analoger Abdruck mit Hilfe eines 3D-Laborscanners digitalisiert wird.

Bei der analogen Abformung von Zähnen und Weichgewebe können verschiedene Abformmassen verwendet werden. Man unterscheidet im Allgemeinen zwischen starren Abformmassen (Abdruckgips, Zinkoxid-Eugenol-Pasten, Kunststoffabformmassen), reversibel starren Abformmassen (Abformwachse, Guttapercha, Kompositionsabdruckmassen) und elastischen Abformmassen. Letztere sind wiederum in irreversibel elastische Abformmassen (Alginate, Polysulfide, Polyether, Silikone) und reversibel elastische Abformmassen, wie Hydrokolloide, unterteilt.

Die analogen Abformverfahren können weiterhin in ringgestützte Abformungen mittels eines sogenannten Kupferring-Kerr-Abdrucks und den integrierten Gesamtabdruck unterteilt werden. Eine Variante der ringgestützen Abformung ist die Sammelabformung, welche sich wie folgt aufgliedert:
1. Trockenlegen des präparierten Zahnstumpfes;
2. Isolieren des Zahnstumpfes mit Vaseline;
3. Entfetten eines zuvor an dem Zahnstumpf feinadjustierten Kupferrings;
4. plastisches Erwärmen einer Kerr-Masse;
5. Einschieben der plastischen Kerr-Masse in den Kupferring;
6. Aufsetzen auf den Zahnstumpf;
7. Abziehen von dem Zahnstumpf und Kontrolle;

Die Schritte 1. bis 7. können für mehrere bzw. sämtliche Zahnstümpfe durchgeführt werden.
8. Wiederaufsetzen sämtlicher Kupferringabformungen auf die Zahnstümpfe;
9. Herstellen eines Sammelabdrucks mit einem elastischen Material; und
10. Herstellen eines Zahnmodells, bei dem die Kupferringe mit einem speziellen Stumpfmaterial ausgegossen wurden, wobei das Gesamtmodell aus Gips gefertigt wird.

Bei der Anfertigung eines integrierten Gesamtabdrucks werden sowohl die Einzelstümpfe als auch der gesamte Kiefer in einem gemeinsamen Abformvorgang erfasst. Sollten die Präparationen der Zähne subgingival sein, muss der Zahnfleischsaum durch geeignete Maßnahmen, wie zum Beispiel mittels Retraktionsfäden, erweitert werden, damit die Abformmasse auch in diese Bereiche vordringen kann.

Aus dem Stand der Technik sind darüber hinaus digitale dreidimensionale Abformverfahren unter Verwendung von intraoralen 3D-Scannern bekannt, wobei derzeit alle am Markt befindlichen Systeme mit lichtoptischen Erfassungstechnologien arbeiten. Beispielsweise sind Systeme bekannt, die auf einem Triangulationsverfahren oder auf konfokaler Abbildung beruhen. Darüber hinaus sind auch andere physikalische Erfassungsmethoden in der Diskussion, beispielsweise die Sonographie, die Erfassung mittels Magnetresonanztomographie, mittels Computertomographie oder mittels digitaler Volumentomographie. Allerdings hat bisher kein derartiges System Marktreife erlangt.

Beim Abdruckscan wird in einem ersten Schritt eine klassische analoge Abformung angefertigt, welche in einem darauffolgenden Arbeitsschritt jedoch nicht mit Gips ausgegossen, sondern mit Hilfe eines Laborscanners digitalisiert wird. Derartige Laborscanner arbeiten häufig auf lichtoptischer Basis, unter Verwendung von Laserlichtschnittverfahren, Streifenlichtprojektion oder konoskopischer Holographie. Sämtliche lichtoptischen Systeme haben jedoch den Nachteil, dass hinterschnittige Bereiche im Abdruck nicht erfasst werden können.

Analoge Abdruckverfahren haben weiterhin den Nachteil, dass sie mitunter einen Würgereiz bei der Abdrucknahme oder ein Gefühl der Hilflosigkeit beim Patienten hervorrufen. Darüber hinaus wird häufig der unangenehme Geschmack der Abformmasse bemängelt.

Die Nachteile der digitalen Abformung bestehen insbesondere darin, dass subgingivale Bereiche von präparierten Zähnen nur unzulänglich erfasst werden können, da diese oftmals durch die Gingivae oder durch Speichel verdeckt sind.

Beim Abdruckscan treten sowohl die Nachteile des analogen als auch die Nachteile des digitalen Abformens auf.

Die US 3,304,608 A beschreibt eine zweiteilig ausgebildete Abformhülse, bei der eine erste, einseitig geschlossene und mit einer Abformmasse befüllte Hülse zur Erzeugung des Abdrucks eines präparierten Zahnes in eine zweite, bereits über den abzuformenden Zahn gestülpte, hohlzylindrische und beidseitig geöffnete Hülse eingeführt wird, wobei der Innendurchmesser der zweiten Hülse im wesentlichen dem Außendurchmesser der ersten Hülse entspricht. Die zweite Hülse weist an ihrem distalen Ende Durchtrittsöffnungen auf, über welche überschüssige Abformmasse austreten kann.

Die DE 10 2009 044 147 A1 beschreibt darüber hinaus ein Verfahren zur Erstellung eines digitalen Datenmodells für zumindest einen Teil eines Oberkiefers, bei dem zumindest ein digitales Oberkieferdatenmodell durch Einscannen zumindest eines Teils des Oberkiefers oder eines Oberkiefermodells oder zumindest eines Teils einer Abformung des Oberkiefermodells oder des Oberkiefermodells mittels einer Scaneinrichtung erzeugt wird. Das Verfahren sieht vor, dass zusätzlich zumindest ein Abdruck von einem Zahn oder von mehreren Zähnen des Oberkiefers in einem Gesichtsbogenregistrat eines Gebissbogens in seiner relativen Lage zu einem dem Gesichtsbogen zugeordneten Koordinatensystem mittels der Scaneinrichtung eingescannt und daraus ein digitales Abdruckdatenmodell erzeugt wird. Anschließend wird die Lage des digitalen Oberkieferdatenmodells in dem dem Gesichtsbogen zugeordneten Koordinatensystem durch Vergleich des digitalen Oberkieferdatenmodells mit dem digitalen Abdruckdatenmodell festgelegt.

### ZUSAMMENFASSUNG DER ERFINDUNG

Es ist die Aufgabe der Erfindung, eine gattungsgemäße Vorrichtung und ein Verfahren zur dreidimensionalen Erfassung von intraoralen Strukturen vorzuschlagen, die einerseits patientenfreundlich sind und andererseits die präzise dreidimensionale Erfassung von intraoralen Strukturen, insbesondere von Zähnen und beschliffenen Zahnstümpfen, erlauben.

Diese Aufgabe wird erfindungsgemäß durch eine Abformvorrichtung gemäß Patentanspruch 1, eine entsprechende extraorale Einscanvorrichtung für das Einscannen einer solchen Abformvorrichtung nach Anspruch 7 und ein entsprechendes Verfahren nach Anspruch 11 gelöst. Alternativ wird sie durch ein Verfahren nach Anspruch 14 gelöst. Die abhängigen Ansprüche betreffen jeweils bevorzugte Ausführungsformen.

Bei der erfindungsgemäßen Abformvorrichtung ist vorgesehen, dass diese ein Abformelement aufweist, das auf einen Zahn oder einen Zahnstumpf aufsetzbar ist, um einen Abdruck des Zahns oder des Zahnstumpfes zu nehmen. Weiterhin umfasst das Abformelement mindestens einen Marker, der so an dem Abformelement angeordnet ist, dass die Position und die Ausrichtung des auf den Zahn oder den Zahnstumpf aufgesetzten Abformelements bei einem Intraoralscan ermittelt werden kann. Die Abformvorrichtung weist in einer Ausführungsform mindestens einen Referenzierungsmarker auf. Alternativ ist sie in einer anderen Ausführungsform der Erfindung mit mindestens einem Referenzierungsmarker verbindbar. Der Referenzierungsmarker ist dabei jeweils so angeordnet, dass er gemeinsam mit dem Abdruck in einem extraoralen Scan abgetastet werden kann.

Mit Hilfe der erfindungsgemäßen Abformvorrichtung ist es möglich, auch diejenigen Bereiche des Mundraumes zu digitalisieren, die bei einem Intraoralscan nach dem Stand der Technik nicht erfasst werden konnten. Dies betrifft insbesondere die subgingivalen Strukturen. Erfindungsgemäß ist ein zweischrittiger Scanvorgang vorgesehen. In einem ersten Schritt wird bei auf dem Zahn oder dem Zahnstumpf aufgesetzter Abformvorrichtung der Mundraum einschließlich der Abformvorrichtung eingescannt. Während dieses Intraoralscans wird auch der Marker der Abformvorrichtung eingescannt, welcher in einer vorbekannten Position relativ zu der Abformvorrichtung angeordnet ist. Das Ergebnis des Intraoralscans ist ein erster Datensatz, der 3D-Informationen des größten Teils des Mundraums und der Position und der Ausrichtung der Abformvorrichtung im Mundraum enthält, nicht jedoch bezüglich der Strukturen, die von der Abformvorrichtung abgedeckt sind. Diese Strukturen werden jedoch gerade analog durch den Abdruck aufgenommen. Folglich ist nach dem Intraoralscan bereits die gesamte 3D-Information aufgenommen, sie liegt jedoch noch nicht vollständig in digitaler Form vor.

Die fehlende digitale Information wird in einem zweiten Schritt mit Hilfe eines Extraoralscans des Abdrucks gewonnen. Der dabei gewonnene Datensatz enthält genau diejenige 3D-Information, die im ersten Datensatz fehlt. Um einen vollständigen Datensatz, das heißt ein vollständiges digitales 3D-Abbild des Mundraums zu erhalten, müssen die beiden Datensätze miteinander verbunden werden. Dies wird im Stand der Technik üblicherweise als "Registrieren" bezeichnet. Das Registrieren wird unter Zuhilfenahme des Referenzierungsmarkers erreicht, der gemeinsam mit dem Extraoralscan des Abdrucks abgetastet wird und dessen Position in Bezug auf den Marker der Abformvorrichtung, und dadurch in Bezug auf den ersten Datensatz, bekannt ist.

Wie bereits angeführt kann der Referenzierungsmarker je nach Ausführungsform entweder Teil der Abformvorrichtung oder mit der Abformvorrichtung verbindbar sein. So ist es beispielsweise denkbar, dass der Referenzierungsmarker an einer Außenseite der Abformvorrichtung angebracht ist, in einer Weise, dass er einerseits bei dem Extraoralscan erfasst werden kann, andererseits jedoch auch nicht bei dem Intraoralscan wesentliche Bereiche des Mundraums überdeckt, welche während des Intraoralscans erfasst werden sollen.

Bei einer anderen Ausführungsform ist vorgesehen, dass der Referenzierungsmarker Bestandteil der extraoralen Einscanvorrichtung ist, wobei der Referenzierungsmarker wiederum derart an der extraoralen Einscanvorrichtung angeordnet ist, dass er gemeinsam mit dem Abdruck in der extraoralen Einscanvorrichtung abgetastet werden kann. Bei dieser Ausführungsform muss demnach das Abformelement selbst keinen Referenzierungsmarker aufweisen, der bei dem Extraoralscan mit erfasst wird. Jedoch ist der Referenzierungsmarker in dieser Ausführungsform mit der Abformvorrichtung "verbindbar", nämlich über die extraorale Einscanvorrichtung. In diesem breiten Sinne ist in der vorliegenden Offenbarung der Begriff "verbindbar" zu verstehen. Bei dieser Ausführungsform ist es wesentlich, dass das Abformelement für den Extraoralscan exakt in der extraoralen Einscanvorrichtung positioniert wird, zumindest jedoch exakt in Bezug auf den Referenzierungsmarker. Die im Folgenden noch näher beschriebenen Positionierungsmittel zur räumlich exakten Positionierung des Abformelements in Bezug auf die extraorale Einscanvorrichtung haben sich dafür als besonders zweckmäßig herausgestellt.

Während Ausführungsformen, bei denen der Referenzierungsmarker Bestandteil der Abformvorrichtung ist, den Vorteil haben, dass die räumliche Anordnung des Referenzierungsmarkers in Bezug auf die Abformvorrichtung mit höchster Präzision vorbestimmt ist, haben Ausführungsformen, bei denen der Referenzierungsmarker an der extraoralen Einscanvorrichtung vorgesehen ist, den Vorteil, dass der Referenzierungsmarker an der Abformvorrichtung entfallen kann, wodurch die Abformvorrichtung technisch einfacher ausfällt und damit preisgünstiger herzustellen ist. Im Übrigen besteht bei einem Referenzierungsmarker, der direkt an dem Abformelement angeordnet ist, die Gefahr, dass der Referenzierungsmarker während des Intraoralscans wesentliche Bereiche des Mundraumes überdeckt und damit unscanbar macht. Dieses Problem lässt sich bei einem Referenzierungsmarker, der mit der Abformvorrichtung nicht dauerhaft verbunden, sondern lediglich zum Zwecke des Extraoralscans "verbindbar" ist, umgehen.

Ein wesentlicher Grundgedanke der Erfindung besteht somit darin, für das Registrieren zweier Datensätze einen Marker und einen Referenzierungsmarker vorzusehen, die jeweils eine bekannte Beziehung zu einem Abformelement aufweisen, das sowohl in einem Intraoralscan als auch in einem Extraoralscan erfasst wird, so dass über die bekannte Beziehung des Markers beziehungsweise des Referenzierungsmarkers zu der Abformvorrichtung das Registrieren des Datensatzes, der bei dem Intraoralscan gewonnen wird, mit dem Datensatz, der bei dem Extraoralscan gewonnen wird, möglich ist. Folglich soll erfindungsgemäß auch die Anordnung des Markers beziehungsweise des Referenzierungsmarkers in Bezug auf die Abformvorrichtung nicht auf die hierin beschriebenen Beispiele beschränkt sein.

Eine Ausführungsform der Erfindung weist als Abformelement eine Hülse zur Aufnahme einer Abformmasse auf. Die Hülse weist dabei ein offenes und ein geschlossenes Ende auf, wobei der Marker an dem geschlossenen Ende angeordnet ist.

Der Marker kann beispielsweise zumindest abschnittsweise kugelförmig ausgebildet sein. Darüber hinaus kann der Marker mindestens eine seitliche Abflachung aufweisen, die je nach Ausführungsform zumindest abschnittsweise eben ausgebildet ist. Bei einer besonders bevorzugten Ausführungsform weist der Marker zumindest zwei Abflachungen auf, die zumindest abschnittsweise in senkrecht zueinander liegenden Ebenen angeordnet sind. Für besonders präzise Scannergebnisse ist es bevorzugt, dass der Marker mehrere der vorgenannten Merkmale kombiniert. Ein besonders effektiver Marker ist daher zumindest abschnittsweise kugelförmig ausgebildet und umfasst zumindest zwei ebene Abflachungen, die zumindest abschnittweise in senkrecht zueinander liegenden Ebenen angeordnet sind.

Bei einer anderen Ausführungsform ist der Marker zumindest abschnittsweise pyramidenförmig ausgebildet. Dem pyramidenförmigen Abschnitt des Markers kann eine Pyramide mit dreieckiger Grundfläche, mit quadratischer Grundfläche, oder mit rechteckiger Grundfläche zugrunde liegen.

Bei einer alternativen Ausführungsform weist der Marker mindestens ein kugelförmiges Element auf. So ist es beispielsweise denkbar, dass der Marker vier halbkugelförmige Elemente aufweist, die bevorzugt in einer gemeinsamen Ebene an den Eckpunkten eines Quadrates oder eines Rechtecks angeordnet sind.

Eine weitere Ausführungsform der Erfindung sieht vor, dass der Marker mindestens zwei übereinander angeordnete Kugeln aufweist, wobei mindestens eine der Kugeln mindestens eine Abflachung aufweist. Besonders bevorzugt weist jede der Kugeln zumindest zwei Abflachungen auf, wobei je Kugel zwei Abflachungen zumindest abschnittsweise eben ausgebildet und senkrecht zueinander ausgerichtet sind.

Es ist weiterhin bevorzugt, dass der Marker über ein Verbindungselement mit der Abformhülse verbunden ist, wobei sich das Verbindungselement von dem geschlossenen Ende der Abformhülse erstreckt. Vorzugsweise weist das Verbindungselement eine Verbindungsfläche auf, über die der mindestens eine Marker mit dem Verbindungselement in Kontakt steht. Dabei kann sich die Verbindungsfläche parallel zu dem geschlossenen Ende der Abformhülse erstrecken.

Es ist bevorzugt, dass das Verbindungselement eine weitere Funktion aufweist, wonach es Positionierungsmittel zur räumlich exakten Positionierung der Abformvorrichtung in einer extraoralen Einscanvorrichtung beziehungsweise in einem Halter derselben aufweist oder bildet. Die Positionierungsmittel sind im Rahmen der Erfindung in keiner Weise beschränkt. Um ein einfaches Einsetzen der Abformvorrichtung in beziehungsweise Entnehmen der Abformvorrichtung aus der Einscanvorrichtung heraus zu ermöglichen, weist das Positionierungsmittel mindestens eine Nut oder eine Feder einer Nut-Feder-Verbindung auf. Besonders vorzugsweise ist zwischen der extraoralen Einscanvorrichtung beziehungsweise dem Halter und der Abformvorrichtung über das Verbindungselement mit Hilfe des Positionierungsmittels zur räumlich exakten Positionierung eine formschlüssige Schwalbenschwanzverbindung ausgebildet. Diese hat den Vorteil, dass die Abformvorrichtung zu ihrer Befestigung in der extraoralen Einscanvorrichtung entlang der Symmetrieachse der Nut-Feder-Verbindung linear verschoben werden kann.

In einer vorteilhaften Weiterbildung umfasst (auch) der Marker ein Positionierungsmittel zur räumlich exakten Positionierung der Abformvorrichtung in einer extraoralen Einscanvorrichtung. Dieses Mittel kann beispielsweise eine Führungsnut sein, die an einem von der Abformhülse abgewandten Ende des Markers ausgebildet ist. Die Führungsnut des Markers kann beim Einsetzen des Abformelements in eine extraorale Einscanvorrichtung mit einer Führungsrippe der extraoralen Einscanvorrichtung in Eingriff gebracht werden. Zweckmäßigerweise ist die Symmetrieachse der Führungsnut parallel zu der Symmetrieachse der Nut-Feder-Verbindung, sofern vorgesehen, ausgerichtet.

Vorzugsweise sind die Mittel für die räumlich exakte Positionierung der Abformvorrichtung in der extraoralen Einscanvorrichtung derart gegenstückig ausgebildet, dass wann immer die Abformvorrichtung oder die extraorale Einscanvorrichtung ein erstes Mittel für die räumlich exakte Positionierung der Abformvorrichtung aufweist, die jeweils andere Baugruppe ein das erste Mittel ergänzendes Mittel aufweisen soll, so dass durch deren Zusammenwirken die räumlich exakte Positionierung der Abformvorrichtung in einer extraoralen Einscanvorrichtung erreicht wird. Beispielhaft sei für solche zusammenwirkenden Mittel die bereits erwähnte Nut-Feder-Verbindung genannt, bei der eine der beiden Baugruppen (d. h. Abformvorrichtung oder extraorale Einscanvorrichtung) die Nut und das jeweils andere die Feder aufweist. Ebenso ist es denkbar, dass die extraorale Einscanvorrichtung beziehungsweise der Halter entweder eine Führungsrippe oder eine Führungsnut aufweist, die mit der jeweils anderen Baugruppe, welche an dem von der Abformhülse abgewandten Ende des Markers ausgebildet ist, in Eingriff bringbar ist.

Das der Erfindung zugrundeliegende Verfahren zur dreidimensionalen Erfassung von intraoralen Strukturen, insbesondere Zähnen und beschliffenen Zahnstümpfen, umfasst die folgenden Schritte:
- Bereitstellen einer wie oben beschrieben ausgestalteten Abformvorrichtung, wobei das Abformelement der Abformvorrichtung mit einer Abformmasse befüllt ist;
- Aufsetzen der Abformvorrichtung auf einen Zahn oder einen Zahnstumpf innerhalb eines Mundraumes, wobei in der Abformmasse ein analoger Abdruck des Zahnes oder des Zahnstumpfes hergestellt wird;
- Abtasten des Mundraumes einschließlich des an dem Abformelement angeordneten Markers mit einem Intraoralscanner und Erzeugen eines ersten Datensatzes, der ein digitalisiertes, dreidimensionales Abbild des Mundraums und des Markers umfasst;
- Entnehmen der Abformvorrichtung aus dem Mundraum und Abtasten des analogen Abdrucks sowie des Referenzierungsmarkers und Erzeugen eines zweiten Datensatzes, der ein digitalisiertes, dreidimensionales Abbild des analogen Abdrucks sowie des Referenzierungsmarkers umfasst.

Wenn für das Abtasten des analogen Abdrucks sowie des Referenzierungsmarkers eine extraorale Einscanvorrichtung verwendet werden soll, kann die Abformvorrichtung dazu in eine Einscanvorrichtung nach einer der oben beschriebenen Ausführungsformen eingesetzt werden, wobei auf die räumlich exakte Anordnung der Abformvorrichtung insbesondere in Bezug auf den Referenzierungsmarker der Einscanvorrichtung zu achten ist.

Dabei ist es bevorzugt, dass das erfindungsgemäße Verfahren im Anschluss an die vorgenannten Schritte das Zusammenlegen des ersten und des zweiten Datensatzes unter Heranziehung einer bekannten räumlichen Beziehung zwischen dem mindestens einen Marker der Abformvorrichtung und dem Referenzierungsmarker umfasst.

In einer alternativen Ausführungsform kann auf den Marker der Abformvorrichtung und den Referenzierungsmarker verzichtet werden und die Registrierung allein anhand der aufgezeichneten dreidimensionalen Daten vorgenommen werden. Das Verfahren in dieser Ausführungsform der Erfindung umfasst die folgenden Schritte:
A.) Bereitstellen einer Abformvorrichtung, umfassend oder bestehend aus einem Abformelement, das eine Abformmasse enthält und auf einen Zahn oder einen Zahnstumpf aufsetzbar ist, um einen Abdruck eines Zahns oder eines Zahnstumpfes zu nehmen,
B.) Aufsetzen der Abformvorrichtung auf einen Zahn oder einen Zahnstumpf innerhalb eines Mundraums, wobei in der Abformmasse ein analoger Abdruck des Zahns oder Zahnstumpfes hergestellt wird,
C.) Entnehmen der Abformvorrichtung aus dem Mundraum,
D.) Vor Schritt B.) oder nach Schritt C.), Abtasten des Mundraums mit einem Intraoralscanner und Erzeugen eines ersten Datensatzes, der ein digitales Abbild des Mundraums ohne die aufgesetzte Abformvorrichtung umfasst,
E.) Abtasten des analogen Abdrucks und Erzeugen eines zweiten Datensatzes der ein digitalisiertes dreidimensionales Abbild des analogen Abdrucks umfasst, und
F.) Registrieren des ersten und des zweiten Datensatzes, indem ein Abschnitt des digitalen Abbildes des Zahnes oder des Zahnstumpfes im ersten Datensatz mit einem Abschnitt des digitalen Abbildes des analogen Abdrucks im zweiten Datensatz in Deckung gebracht wird.

Abweichend von der oben beschriebenen Ausführungsform wird in dieser alternativen Ausführungsform der Intraoralscan ohne aufgesetzte Abformvorrichtung durchgeführt. Stattdessen wird der Zahn oder der Zahnstumpf direkt mittels Intraoralscan gescannt. Wie eingangs erwähnt, tritt dabei grundsätzlich das Problem auf, dass die subgingivalen Strukturen nicht erfasst werden können. Zu diesem Zweck wird jedoch zusätzlich auf die oben beschriebene Weise ein analoger Abdruck des Zahns oder des Zahnstumpfes genommen, der dann im oben genannten Schritt E.) abgetastet wird, wodurch ein zweiter Datensatz erzeugt wird, der die subgingivalen Strukturen auf dem Umweg über den analogen Abdruck erfasst. In dieser Ausführungsform werden die beiden Datensätze dadurch registriert, dass gemäß Schritt F.) ein Abschnitt des digitalen Abbildes des Zahns oder des Zahnstumpfes im ersten Datensatz mit einem Abschnitt des digitalen Abbildes des analogen Abdrucks im zweiten Datensatz in Deckung gebracht wird. Für diese Zwecke eignet sich das sogenannte "Best-Fit-Alignment". Das Best-Fit-Alignment ist eine etablierte Methode, Bilder in Übereinstimmung zu bringen. Grob gesprochen handelt es sich dabei um ein Minimierungsverfahren, bei dem die Summe der Abstände zwischen korrespondierenden Punkten an den in Deckung zu bringenden Objekten minimiert wird.

Der grundsätzliche Gedanke, einen Mundraumscan und einen Scan eines analogen Abdrucks zu vereinen, kommt auch in dieser Ausführungsform zum Tragen. Der Unterschied zu der vorbeschriebenen Ausfihrungsform besteht darin, dass die Registrierung direkt an den Strukturen der Datensätze vorgenommen wird, statt einen Umweg über korrespondierende Marker in den beiden Datensätzen zu gehen.

Vorzugsweise umfasst das Zusammenlegen des ersten und des zweiten Datensatzes ein Best-Fit-Alignment.

### KURZBESCHREIBUNG DER FIGUREN

Weitere Einzelheiten der Erfindung werden anhand der nachstehenden Figuren erläutert. Dabei zeigt:
- Figur 1: eine Querschnittsansicht einer ersten Ausführungsform der erfindungsgemäßen Abformvorrichtung;
- Figur 2: eine Draufsicht auf die Abformvorrichtung gemäß Figur 1;
- Figur 3: eine Querschnittsansicht einer zweiten Ausführungsform der erfindungsgemäßen Abformvorrichtung;
- Figur 4: eine Draufsicht auf die Abformvorrichtung gemäß Figur 3;
- Figur 5: eine Querschnittsansicht einer dritten Ausführungsform der erfindungsgemäßen Abformvorrichtung;
- Figur 6: eine Draufsicht auf die Abformvorrichtung gemäß Figur 5;
- Figur 7: eine Querschnittsansicht einer vierten Ausführungsform der erfindungsgemäßen Abformvorrichtung;
- Figur 8: eine Draufsicht auf die Abformvorrichtung gemäß Figur 7;
- Figur 9: eine Querschnittsansicht einer extraoralen Einscanvorrichtung gemäß einer ersten Ausführungsform mit einer eingesetzten Abformvorrichtung gemäß den Figuren 1 und 2;
- Figur 10: eine Draufsicht auf die extraorale Einscanvorrichtung gemäß Figur 9;
- Figur 11: eine Seitenansicht eines Abformelementes, an dem ein geometrischer Referenzierungsmarker vorgesehen ist;
- Figur 12: eine Draufsicht auf das Abformelement von Fig. 11, d.h. mit Blick auf den Abdruck; und
- Figur 13: eine Ansicht wie in Fig. 12, jedoch mit einem optischen Marker anstatt eines geometrischen Markers.

### BESCHREIBUNG DER BEVORZUGTEN AUSFÜHRUNGSFORMEN

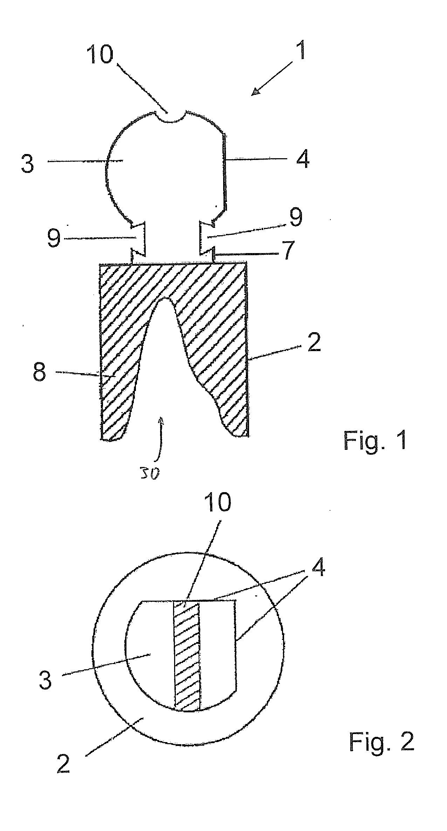

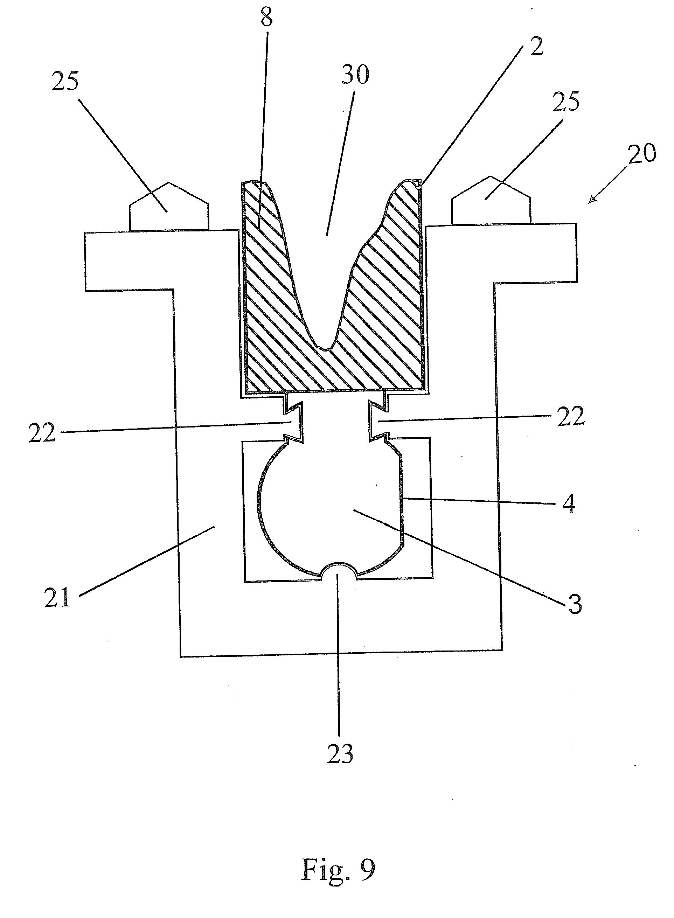

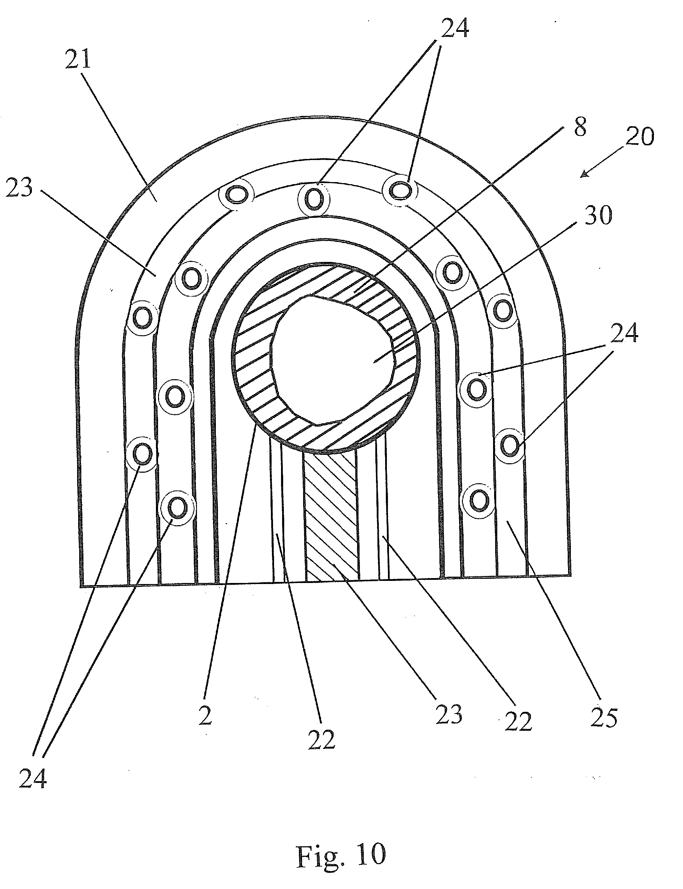

In der Figur 1 ist eine erste Ausführungsform der erfindungsgemäßen Abformvorrichtung 1 dargestellt, mit einem als Abformhülse 2 ausgebildeten Abformelement, das mit einer Abformmasse 8 befüllt ist. Die Abformhülse 2 weist ein offenes und ein geschlossenes Ende auf sowie einen Marker 3, der über ein Verbindungselement 7 mit der Abformhülse 2 verbunden ist, wobei sich das Verbindungselement 7 von dem geschlossenen Ende der Abformhülse 2 erstreckt. Darüber hinaus ist ein in der Abformmasse 8 abgebildeter Abdruck 30 zu erkennen. Der Marker 3 ist zumindest abschnittsweise kugelförmig ausgebildet und weist in der dargestellten Querschnittsansicht zumindest eine erkennbare Abflachung 4 auf. Wie in der Figur 2 zu erkennen ist, weist der Marker 3 neben der in Figur gezeigten Abflachung 4 eine weitere Abflachung 4 auf, wobei die Abflachungen 4 senkrecht zueinander ausgerichtet und jeweils eben ausgebildet sind. Das Verbindungselement 7 weist Mittel 9 zur räumlich exakten Positionierung der Abformvorrichtung 1 in einer extraoralen Einscanvorrichtung 20 gemäß den Figuren 9 und 10 auf. Die Positionierungsmittel 9 sind als Nuten einer Schwalbenschwanzverbindung ausgebildet und weisen eine Längsachse auf, die senkrecht zur Zeichenebene ausgerichtet ist. Die Nuten 9 können mit entsprechenden Federn, welche Bestandteil einer extraoralen Einscanvorrichtung 20 (siehe Figuren 9 und 10) oder eines Halters derselben sind, in Eingriff gebracht werden, wodurch eine Nut-Feder-Verbindung zur räumlich exakten Positionierung der Abformvorrichtung 1 in der extraoralen Einscanvorrichtung 20 gebildet wird.

Die dargestellte Ausführungsform weist keinen Referenzierungsmarker auf, so dass dieser folglich von einer extraoralen Einscanvorrichtung 20 bereitgestellt werden muss. Aufgrund der bekannten Position und Ausrichtung des Markers 3 in Bezug auf die Abformhülse 2 ist aus dem beim Intraoralscan gewonnenen ersten Datensatz die exakte Position und Ausrichtung des Abformelements 1 im Mundraum bestimmbar. Beim Extraoralscan wird darüber hinaus aus dem dabei gewonnen zweiten Datensatz unter Einbeziehung des Referenzierungsmarkers die Gestalt sowie die Lage und die Ausrichtung des abgeformten Zahnes beziehungsweise Zahnstumpfes in Bezug auf die Abformvorrichtung 1 bestimmt. Schließlich ist nach Registrierung beider Datensätze neben der genauen Gestalt des Zahnes beziehungsweise Zahnstumpfes einschließlich der subgingivalen Strukturen auch seine genaue Position und Ausrichtung innerhalb des Mundraumes bestimmbar. Die Abflachung 4 an dem ansonsten sphärischen Marker 3 kann insbesondere dazu dienen, die Ausrichtung des Zahnes beziehungsweise des Zahnstumpfes zu bestimmen.

In der Figur 2 ist darüber hinaus das weitere Mittel zur räumlich exakten Positionierung der Abformvorrichtung 1 in einer extraoralen Einscanvorrichtung 20 deutlicher zu erkennen, welches vorliegend eine Führungsnut 10 ist, die an einem von der Abformhülse 2 abgewandten Ende des Markers 3 ausgebildet ist.

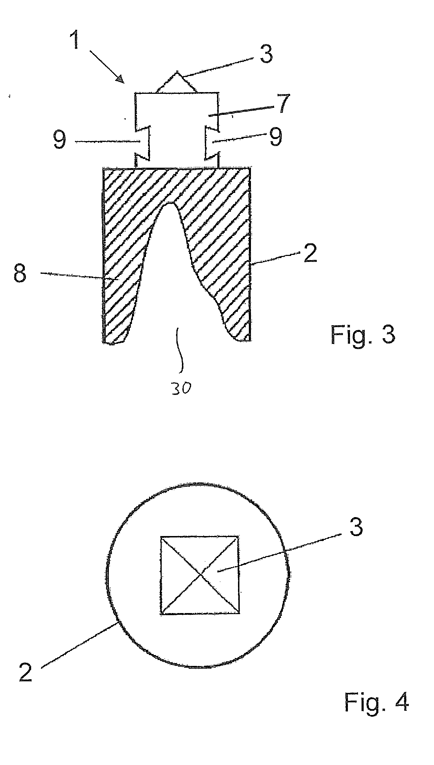

Die in den Figuren 3 und 4 dargestellte Ausführungsform einer erfindungsgemäßen Abformvorrichtung 1 unterscheidet sich von der in den Figuren 1 und 2 gezeigten dadurch, dass der

Marker 3 in Form einer Pyramide mit quadratischer Grundfläche ausgebildet ist. Diese ist wiederum über ein Verbindungselement 7 mit der Abformhülse 2 verbunden, wobei sich das Verbindungselement 7 von dem geschlossenen Ende der Abformhülse 2 erstreckt und entsprechend der vorbeschriebenen Ausführungsform die Positionierungsmittel 9 umfasst.

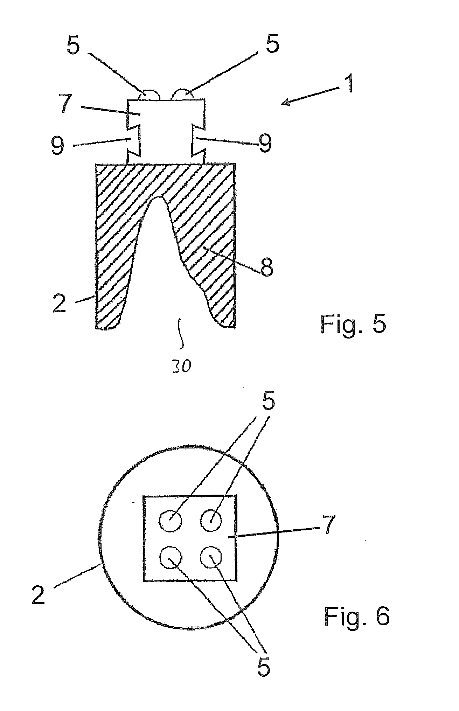

Die in den Figuren 5 und 6 gezeigte Ausführungsform einer erfindungsgemäßen Abformvorrichtung 1 weist als Marker 3 vier halbkugelförmige Elemente 5 auf, die an den Eckpunkten eines Quadrates angeordnet sind.

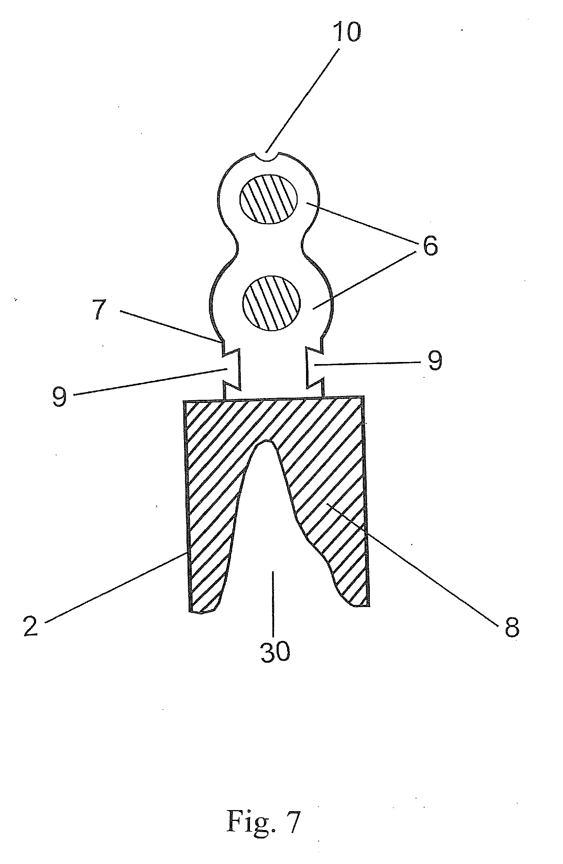

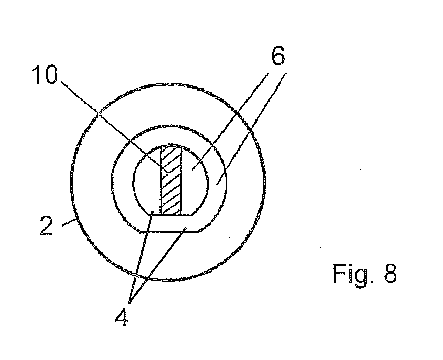

Der Marker 3 der Ausführungsform gemäß den Figuren 7 und 8 umfasst zwei übereinander angeordnete Kugeln 6, wobei jede der Kugeln 6 genau eine Abflachung 4 aufweist, die parallel zueinander angeordnet sind. Die übereinander angeordneten Kugeln 6 des Markers 3 sind wiederum über ein Verbindungselement 7, das an die untere Kugel 6 angrenzt, mit dem geschlossenen Ende der Abformhülse 2 verbunden. Das Verbindungselement 7 umfasst wie bei den vorangegangenen Ausführungsformen wiederum Mittel 9 zur räumlich exakten Positionierung der Abformvorrichtung 1 in einer extraoralen Einscanvorrichtung 20 gemäß den Figuren 9 und 10, wobei die Positionierungsmittel 9 als Nuten einer Schwalbenschwanzverbindung ausgebildet sind. Der Marker 3 umfasst ein weiteres Mittel zur räumlich exakten Positionierung, welches als eine Führungsnut 10 ausgebildet ist, die an dem von der Abformhülse 2 abgewandten Ende des Markers 3 ausgebildet ist und mit einer Führungsrippe 23 (siehe Figuren 9 und 10) in Eingriff bringbar ist, die von der extraoralen Einscanvorrichtung 20 beziehungsweise einem Halter derselben bereitgestellt wird.

In den Figuren 9 und 10 ist eine Querschnittsansicht beziehungsweise eine Draufsicht auf eine erfindungsgemäße extraorale Einscanvorrichtung 20 gezeigt, in welche eine Abformvorrichtung 1 gemäß der Ausführungsform von den Figuren 1 und 2 eingeschoben ist. Die extraorale Einscanvorrichtung 20 umfasst einen Halter 21 in dem die Abformvorrichtung 1 aufgenommen ist. Der Halter 21 umfasst wie auch die Abformvorrichtung 1 Mittel 22 für die räumlich exakte Positionierung der Abformvorrichtung 1 in Bezug auf den Halter 21 und einen Referenzierungsmarker 24. Bei der dargestellten Ausführungsform wirken die Positionierungsmittel 9 (siehe auch Figur 1) der Abformvorrichtung 1 mit den Mitteln 22 des Halters 21 für die räumlich exakte Positionierung der Abformvorrichtung 1 zusammen. Sie bilden eine Schwalbenschwanzverbindung aus. Schließlich umfasst der Halter 21 eine Führungsrippe 23, die in eine Führungsnut 10 (siehe auch Figur 1), welche an dem von der Abformhülse 2 abgewandten Ende des Markers ausgebildet ist, eingreift. Die Referenzierungsmarker 24 sind vorliegend auf einem U-förmigen Referenzierungsring 25 angeordnet.

Die Längsachsen der Mittel 22, der Positionierungsmittel 9, der Führungsnut 10 sowie der Führungsrippe 23 sind parallel ausgerichtet. Dies ermöglicht es, dass die Abformvorrichtung 1 für das räumlich exakte Positionieren in der Einscanvorrichtung 20 linear in die Einscanvorrichtung 20 beziehungsweise den Halter 21 eingeschoben werden kann. Bis auf den Freiheitsgrad in Einschubrichtung ist die Position der Abformvorrichtung 1 in der Einscanvorrichtung 20 somit bereits durch die vorbeschriebenen Mittel vorgegeben. Die exakte Lage in Einschubrichtung kann durch einen Anschlag, eine Rastposition oder ähnliche Mittel vorgegeben werden.

Nach dem Einscannen sowohl des abgenommenen Abdrucks als auch des an der Einscanvorrichtung 20 bereitgestellten Referenzierungsmarkers 24 können aufgrund der bekannten Anordnung von Referenzierungsmarker 24 und Abformvorrichtung 1 zueinander und unter Verwendung der aus dem Intraoralscan gewonnenen Information über die Lage der Abformvorrichtung 1 während des Abdrucks im Mundraum die genaue Lage, Ausrichtung und Geometrie des abgeformten Zahnes beziehungsweise Zahnstumpfes im Mundraum bestimmt werden.

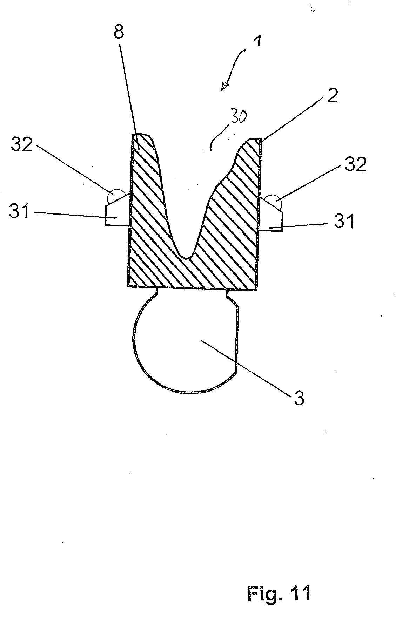

Wie eingangs erwähnt, ist es nicht notwendig, dass der Referenzierungsmarker 24 an der Einscanvorrichtung 20 vorgesehen ist. Stattdessen ist es ebenso möglich, einen Referenzierungsmarker direkt an der Abformvorrichtung 1 vorzusehen, der geeignet ist, in dem extraoralen Scann miterfasst zu werden. Beispiele hierfür sind in Fig. 11, 12 und 13 gezeigt.

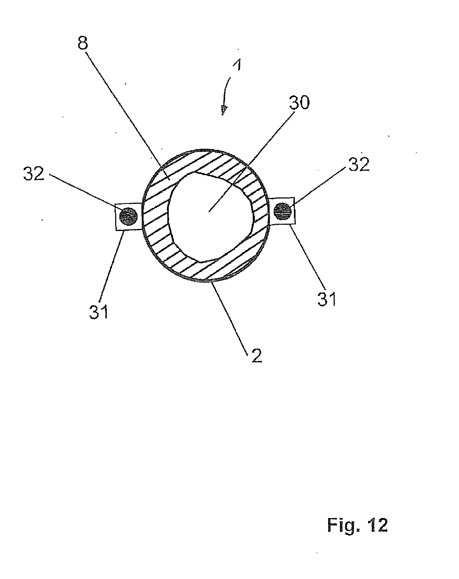

Fig. 11 zeigt eine Schnittansicht einer Abformvorrichtung 1, die grundsätzlich ähnlich aufgebaut ist wie diejenigen aus den oben beschriebenen Ausführungsformen. Zusätzlich zu dem Marker 3 enthält diese Abformvorrichtung 1 jedoch eine Markeranordnung 31, die in dem gezeigten Ausführungsbeispiel einen kugelförmigen Referenzierungsmarker 32 trägt. Da der Referenzierungsmarker 32 sich durch seine dreidimensionale Gestalt auszeichnet, wird er auch als "geometrischer Marker" bezeichnet. Der Referenzierungsmarker 32 ist so angeordnet, dass er beim extraoralen Scan des Abdrucks 30 in der Abformmasse 8 gut miterfasst werden kann. Dies wird besonders aus Fig. 12 deutlich, die eine Draufsicht auf die Abbildung von Fig. 11 zeigt, d. h. einen Blick in den Abdruck 30 in der Hülse 2 hinein. Wie aus Fig. 12 zu sehen ist, ist in dieser Ansicht die Markeranordnung 31 mit dem Referenzierungsmarker 32 gut zu erkennen.

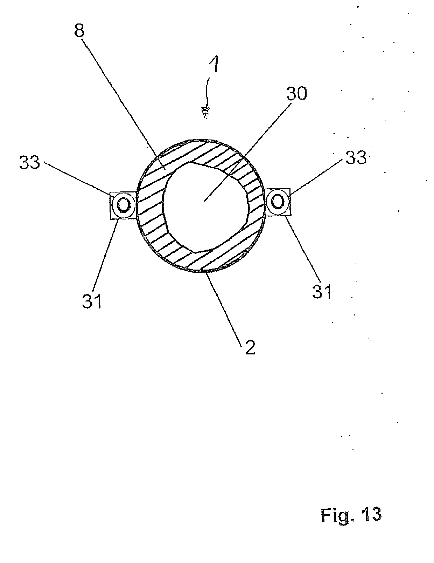

Fig. 13 zeigt eine alternative Ausführungsform, bei der die Markeranordnung 31 keinen geometrischen, sondern einen sogenannten "optischen Marker" 33 zeigt. Ein "optischer" Marker zeichnet sich nicht durch eine bestimmte Geometrie, bzw. dreidimensionale Form, sondern durch eine bestimmte optische Darstellung aus, im gezeigten Ausführungsbeispiel ein dunkler Ring auf einem hellen Kreis. Die Referenzierungsmarker 32 und 33 sind lediglich beispielhaft zu verstehen.

Die Lösung der Erfindung beruht somit darauf, die gesamte Erfassung des Kiefers, der dazugehörigen Zähne und der Gingiva mit Hilfe eines digitalen 3D-Erfassungssystems zu realisieren. Dabei werden von dem behandelnden Arzt in einem ersten Schritt die präparierten Zähne mittels einer Abformhülse analog abgeformt. Im Einzelnen ergeben sich die folgenden Arbeitsschritte:
a) Präparation der Zähne
b) Auswahl der geeigneten Größe der digitalen Abformhülse, wobei diese jeweils in Abhängigkeit des präparierten Zahnes hinsichtlich ihrer Größe und Geometrie ausgewählt werden kann. Eine individuelle Adaption der Abformhülse durch partielles Einkürzen ist ebenfalls denkbar. Die Abformhülse kann entweder aus Kunststoff oder aus einem Metall gefertigt sein. Als Kunststoff hat sich insbesondere PEEK (Polyetheretherketon) bewährt, da dieses Material sehr gut scannbar ist. Für aus Metall gefertigte Abformhülsen hat sich insbesondere Titan als zweckmäßig herausgestellt. Vorzugsweise sind derartige Abformhülsen mit einer Beschichtung versehen, welche die Scannbarkeit der Hülse verbessern.

Die erfindungsgemäße Abformhülse weist eine charakteristische Geometrie auf. Sie umfasst beispielsweise einen im Wesentlichen hohlzylindrischen Aufbau, wobei die Grundfläche vorzugsweise kreisförmig oder oval ausgebildet ist. Idealerweise ist die Grundfläche nach den Querschnitten der verschiedenen Zahngrundtypen ausgewählt. Als Referenzquerschnitt sollte dabei der horizontale Schnitt durch den jeweiligen Zahntyp auf der Höhe des Überganges der Zahnwurzel zur Zahnkrone verwendet werden. Ebenso können für jeden Zahntyp verschiedene Größen und Längen der Hülsen geeignet sein.

Die Geometrie des Markers ist insbesondere danach auszuwählen, dass er mit Hilfe gängiger intraoraler Scanner möglichst geometrietreu erfasst werden kann.

Bei der analogen Abformung wird die Abformhülse mit Abformmasse aufgefüllt und anschließend auf den präparierten Zahn oder Zahnstumpf aufgesetzt. Eventuell seitlich überquellendes Material kann dabei entfernt werden. Die abzuformenden Zähne oder Zahnstümpfe können jeweils einzeln oder gleichzeitig abgeformt werden. Die Abformhülsen bleiben für das intraorale Scannen in situ.

In einem nächsten Schritt erfolgt die digitale 3D-Erfassung mittels Intraoralscanner. Dabei werden alle für die Weiterverarbeitung notwendigen Strukturen erfasst, insbesondere Zähne, die Gingiva, präparierte Stümpfe und Gegenkieferzähne. Die Erfassung von subgingivalen Bereichen der präparierten Zähne oder Zahnstümpfe ist hierbei nicht möglich. Diese wurden jedoch vorher bereits über den analogen Abdruck erfasst. Die scannbare Abformhülse mit ihrem Marker wird in diesem Arbeitsschritt ebenfalls digitalisiert.

Für das extraorale Einscannen mit Hilfe der extraoralen Einscanvorrichtung wird nach dem Aushärten der Abformmasse die Abformvorrichtung von dem präparierten Zahn oder Zahnstumpf gezogen. Anschließend wird die Abformvorrichtung in der extraoralen Einscanvorrichtung positioniert. Dies kann beispielsweise durch seitliches Einschieben erfolgen, bei dem zwischen den Mitteln für die räumlich exakte Positionierung der Abformvorrichtung in der extraoralen Einscanvorrichtung sowohl der Abformvorrichtung als auch der extraoralen Einscanvorrichtung eine Nut-Feder-Verbindung zwischen den Baugruppen hergestellt wird. Diese kann beispielsweise als Schwalbenschwanzverbindung oder als Prismenführung ausgestaltet sein. Vorzugsweise werden an gegenüberliegenden Seiten des Verbindungselements der Abformvorrichtung derartige Verbindungen zwischen der Abformvorrichtung und der extraoralen Einscanvorrichtung beziehungsweise einem Halter derselben ausgebildet. Auf diese Weise kann die Abformvorrichtung durch Einschieben in die extraorale Einscanvorrichtung mit dieser verbunden werden. Dabei kann die Endposition in Einschubrichtung beispielsweise mit Hilfe eines Anschlags festgelegt sein.

In einem nächsten Schritt wird der Zahn- oder Zahnstumpfabdruck in der Abformhülse vorzugsweise lichtoptisch eingescannt, was entweder mit Hilfe eines separaten Scanners erfolgen kann oder mit Hilfe des bereits für den interoralen Scannvorgang verwendeten digitalen 3D-Intraoralscanners.

Durch die Verwendung eines Referenzierungsmarkers, welcher beim extraoralen Einscannen der Abformvorrichtung mit erfasst wird, kann die genaue Position des mit der Abformvorrichtung abgeformten Zahnstumpfes in dem Gesamtbild des Kiefers, welches mit Hilfe des intraoralen Scanners erfasst worden ist, ermittelt werden. Das Ergebnis ist ein digitaler intraoraler Abdruck, der neben einem Scan des gesamten Kiefers auch Scandaten bezüglich der subgingivalen Präparation aufweist.

In einer alternativen Ausführungsform kann auf den Marker 3 der Abformvorrichtung 1 und die Referenzierungsmarker 24, 32 oder 33 verzichtet werden. Stattdessen wird der Intraoralscan des Mundraums ohne Abformvorrichtung 1 aufgenommen, um einen ersten Datensatz zu erzeugen, der ein digitales Abbild des Mundraums umfasst. Davor oder danach (nicht jedoch während des Intraoralscans) wird eine Abformvorrichtung 1 auf einen Zahn oder einen Zahnstumpf innerhalb des Mundraums aufgesetzt, wobei in der Abformmasse 8 ein analoger Abdruck 30 des Zahns oder des Zahnstumpfes hergestellt wird. Die Abformvorrichtung 1 kann grundsätzlich von einer der oben beschriebenen Arten sein, mit der Ausnahme dass sie keinen Marker 3 benötigt.

Der analoge Abdruck 30 in der Abformvorrichtung 1 wird dann extraoral gescannt, um einen zweiten Datensatz zu erhalten. Dieser zweite Datensatz enthält anders als in der oben beschriebenen Ausführungsform keinen Referenzierungsmarker 24, 32 oder 33. Allerdings enthält dieser zweite Datensatz wie zuvor sämtliche Informationen des analogen Abdrucks 30, d.h. auch die subgingivalen Strukturen, die in dem ersten Datensatz aus den oben beschriebenen Gründen nicht abgebildet sind.

In dieser Ausführungsform werden schließlich der erste und der zweite Datensatz dadurch registriert, dass ein Abschnitt des digitalen Abbildes des Zahns oder des Zahnstumpfes in dem ersten Datensatz mit einem Abschnitt des digitalen Abbildes des analogen Abdrucks im zweiten Datensatz in Deckung gebracht wird, beispielsweise mittels eines Best-Fit-Alignments. Eine Besonderheit der Erfindung in beiden beschriebenen Ausführungsformen besteht somit in der Kombination analoger und digitaler Abformtechniken, wobei die jeweiligen Nachteile vermieden und die jeweiligen Vorteile beider Abformtechniken nutzbar gemacht werden. Somit ist es nunmehr möglich, Zahnbereiche digital zu erfassen, welche ansonsten mittels digitaler Abformtechniken nicht erreicht werden können. Dies sind insbesondere die subgingivalen Bereiche von präparierten Zähnen oder Zahnstümpfen.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### BEZUGSZEICHENLISTE

- 1: Abformvorrichtung
- 2: Abformhülse
- 3: Marker
- 4: Abflachung
- 5: Halbkugelförmiges Element
- 6: Kugel
- 7: Verbindungselement
- 8: Abformmasse
- 9: Mittel zur räumlich exakten Positionierung der Abformvorrichtung
- 10: Führungsnut
- 20: Einscanvorrichtung
- 21: Halter
- 22: Mittel für die räumlich exakte Positionierung der Abformvorrichtung
- 23: Führungsrippe
- 24: Referenzierungsmarker
- 25: Referenzierungsring
- 30: Abdruck
- 31: Markeranordnung
- 32: Geometrischer Referenzierungsmarker
- 33: Optischer Referenzierungsmarker

## Patentansprüche

1. Abformvorrichtung (1) zur dreidimensionalen Erfassung von intraoralen Strukturen, mit einem Abformelement (2), das auf einen Zahn oder einen Zahnstumpf aufsetzbar ist, um einen Abdruck des Zahns oder des Zahnstumpfes zu nehmen, und mindestens einem Marker (3), der so an dem Abformelement (2) angeordnet ist, dass die Position und die Ausrichtung des auf den Zahn oder den Zahnstumpf aufgesetzten Abformelementes (2) bei einem Intraoralscan ermittelt werden kann, wobei die Abformvorrichtung (1) mindestens einen Referenzierungsmarker (24) aufweist oder mit einem Referenzierungsmarker (24) verbindbar ist, der so angeordnet ist, dass er gemeinsam mit dem Abdruck in einem extraoralen Scan abgetastet werden kann.

2. Abformvorrichtung (1) nach Anspruch 1, bei der das Abformelement (2) eine Hülse zur Aufnahme einer Abformmasse (8) aufweist, mit einem offenen und einem geschlossenen Ende, wobei der Marker (3) an dem geschlossenen Ende angeordnet ist.

3. Abformvorrichtung (1) nach Anspruch 1 oder 2, bei der der Marker (3)
- zumindest abschnittsweise kugelförmig oder pyramidenförmig ausgebildet ist, und/oder
- mindestens eine, vorzugsweise zumindest abschnittsweise eben ausgebildete, seitliche Abflachung (4) aufweist, und/oder
- mindestens ein halbkugelförmiges Element aufweist, und/oder
- mindestens zwei übereinander angeordnete Kugeln (6) aufweist, wobei mindestens eine der Kugeln (6) mindestens eine Abflachung (4) aufweist.

4. Abformvorrichtung (1) nach Anspruch 2 oder 3, bei der der Marker (3) über ein Verbindungselement (7) mit der Abformhülse (2) verbunden ist, wobei sich das Verbindungselement (7) von dem geschlossenen Ende der Abformhülse (2) erstreckt.

5. Abformvorrichtung (1) nach Anspruch 4, bei der das Verbindungselement (7) Positionierungsmittel (9) zur räumlich exakten Positionierung der Abformvorrichtung (1) in einer extraoralen Einscanvorrichtung aufweist, wobei die Positionierungsmittel (9) vorzugsweise mindestens eine Nut oder eine Feder einer Nut-Feder-Verbindung aufweisen.

6. Abformvorrichtung (1) nach einem der vorangegangenen Ansprüche, bei der der Marker (3) ein Positionierungsmittel (9) zur räumlich exakten Positionierung der Abformvorrichtung (1) in einer extraoralen Einscanvorrichtung (20) aufweist, wobei das Positionierungsmittel (9) des Markers (3) vorzugsweise eine Führungsnut (10) oder eine Führungsrippe (23) aufweist, die an einem von der Abformhülse (2) abgewandten Ende des Markers (3) ausgebildet ist.

7. Extraorale Einscanvorrichtung (20) für das extraorale Einscannen eines Abdrucks eines Zahnes oder eines Zahnstumpfes, der mit Hilfe einer Abformvorrichtung (1) nach einem der Ansprüche 1 bis 8 aufgenommen worden ist, wobei die Einscanvorrichtung (20) Positionierungsmittel (22) für die räumlich exakte Positionierung der Abformvorrichtung (1) in Bezug auf die Einscanvorrichtung (20) aufweist.

8. Extraorale Einscanvorrichtung (20) nach Anspruch 7, **dadurch gekennzeichnet, dass** sie mindestens einen Referenzierungsmarker (24) aufweist, der so angeordnet ist, dass er gemeinsam mit dem Abdruck in einem extraoralen Scan abgetastet werden kann, wenn die Abformvorrichtung (1) in der extraoralen Einscanvorrichtung (20) aufgenommen ist.

9. Extraorale Einscanvorrichtung (20) nach Anspruch 7, **dadurch gekennzeichnet, dass** die Positionierungsmittel (9) der Abformvorrichtung (1) für die räumlich exakte Positionierung der Abformvorrichtung (1) mit den Positionierungsmitteln (22) der Einscanvorrichtung (20) für die räumlich exakte Positionierung der Abformvorrichtung (1) zusammenwirken, wobei die zusammenwirkenden Positionierungsmittel (9, 22) vorzugsweise eine Nut-Feder-Verbindung ausbilden.

10. Extraorale Einscanvorrichtung (20) nach einem der Ansprüche 7 bis 9, **dadurch gekennzeichnet, dass** sie eine Führungsrippe (23) aufweist, die mit einer Führungsnut (10), die an dem von einer Abformhülse (2) abgewandten Ende des Markers (3) ausgebildet ist, in Eingriff bringbar ist.

11. Verfahren zur dreidimensionalen Erfassung von intraoralen Strukturen, mit den Schritten:
- Bereitstellen einer Abformvorrichtung (1) nach einem der Ansprüche 1 bis 8, wobei das Abformelement (2) der Abformvorrichtung (1) mit einer Abformmasse (8) befüllt ist;
- Aufsetzen der Abformvorrichtung (1) auf einen Zahn oder einen Zahnstumpf innerhalb eines Mundraumes, wobei in der Abformmasse (8) ein analoger Abdruck (30) des Zahnes oder des Zahnstumpfes hergestellt wird;
- Abtasten des Mundraumes einschließlich des an der Abformhülse (2) angeordneten Markers (3) mit einem Intraoralscanner und Erzeugen eines ersten Datensatzes, der ein digitalisiertes, dreidimensionales Abbild des Mundraums und des Markers (3) umfasst;
- Entnehmen der Abformvorrichtung (1) aus dem Mundraum und Abtasten des analogen Abdrucks (30) sowie des Referenzierungsmarkers (24) und Erzeugen eines zweiten Datensatzes, der ein digitalisiertes, dreidimensionales Abbild des analogen Abdrucks (30) sowie des Referenzierungsmarkers (24) umfasst.

12. Verfahren nach Anspruch 11, bei dem die Abformvorrichtung (1) für das Abtasten in eine extraorale Einscanvorrichtung (20) nach einem der Ansprüche 9 bis 13 eingesetzt wird.

13. Verfahren nach Anspruch 11 oder 12, bei dem der erste und der zweite Datensatz unter Heranziehung einer bekannten räumlichen Beziehung zwischen dem mindestens einen Marker (3) der Abformvorrichtung (1) und dem Referenzierungsmarker (24) registriert werden.

14. Verfahren zur dreidimensionalen Erfassung von intraoralen Strukturen, mit den folgenden Schritten:
A.) Bereitstellen einer Abformvorrichtung (1), umfassend oder bestehend aus einem Abformelement (2), das eine Abformmasse (8) enthält und auf einen Zahn oder einen Zahnstumpf aufsetzbar ist, um einen Abdruck eines Zahns oder eines Zahnstumpfes zu nehmen,
B.) Aufsetzen der Abformvorrichtung (1) auf einen Zahn oder einen Zahnstumpf innerhalb eines Mundraums, wobei in der Abformmasse (8) ein analoger Abdruck (30) des Zahns oder des Zahnstumpfes hergestellt wird,
C.) Entnehmen der Abformvorrichtung (1) aus dem Mundraum,
D.) Vor Schritt B.) oder nach Schritt C.), Abtasten des Mundraums mit einem Intraoralscanner und Erzeugen eines ersten Datensatzes, der ein digitales Abbild des Mundraums ohne die aufgesetzte Abformvorrichtung (1) umfasst,
E.) Abtasten des analogen Abdrucks (30) und Erzeugen eines zweiten Datensatzes der ein digitalisiertes dreidimensionales Abbild des analogen Abdrucks (30) umfasst, und
F.) Registrieren des ersten und des zweiten Datensatzes, indem ein Abschnitt des digitalen Abbildes des Zahnes oder des Zahnstumpfes im ersten Datensatz mit einem Abschnitt des digitalen Abbildes des analogen Abdrucks im zweiten Datensatz in Deckung gebracht wird.

15. Verfahren nach Anspruch 14, bei dem der Abschnitt des digitalen Abbildes des Zahns oder des Zahnstumpfes aus dem ersten Datensatz durch ein Best-Fit-Alignment mit dem digitalen Abbild des analogen Abdrucks (30) aus dem zweiten Datensatz assoziiert wird.
